# EUROPEAN PATENT SPECIFICATION

(11) **EP 0 730 881 B1**
(45) Date of publication and mention of the grant of the patent: **28.07.2004**
(21) Application number: 96301411.3
(22) Date of filing: 01.03.1996
(51) Int. Cl.: A61M 25/06, A61M 5/31

(54) **Two-component needle housing for catheter introducer assembly**
Zweikomponententyp Nadeleinbaugehäuse für ein Katheteranordnung
Boîtier-aiguille du type à deux components pour assemblage d'introduction d'un cathéter

(30) Priority: 02.03.1995 US 393218
(43) Date of publication of application: 11.09.1996
(73) Proprietor: JOHNSON & JOHNSON MEDICAL, INC., Arlington, Texas 76004-3130 (US)
(72) Inventor: Chang, Joseph Jawshin, Avon, CT 06001 (US); Hillstrand, Mark Richard, Southington, CT 06489 (US); Niedziela, Theodore Leo, Southbury, CT 06488 (US)
(74) Representative: Mercer, Christopher Paul

(56) References cited:
- EP-A- 0 299 936
- EP-A- 0 331 318
- EP-A- 0 578 367
- WO-A-94/23784
- US-A- 4 828 549
- US-A- 5 273 540

## Description

### Background of the Invention

### Field of the Invention

This invention relates to medical devices and, in particular, to vascular access products.

There are essentially two types of catheter introducer assemblies in use today for introducing venous catheters into the vascular system of a patient. The first type is a through-the-needle catheter in which the catheter itself is positioned within a piercing needle that is then used to pierce the skin and emplace the catheter within the vascular system of the patient. The needle is then removed and normally split or otherwise incapacitated for the remainder of the use of the catheter.

The second type of catheter emplacement unit and the type related to the present invention is an over-the-needle catheter in which the catheter rides on the outside of a hollow cannula or needle. The needle is used to pierce the skin of a patient and introduce the catheter into the vascular system of the patient. The catheter is then advanced off the needle into the vein and the needle is withdrawn from the inside of the catheter for disposal.

Over-the-needle catheter emplacement units have a drawback in that once the needle is withdrawn a series of other operations must take place before the catheter is finally attached (i.e., taping and attachment of a fluid supply). During this time, the exposed needle is a hazard to both the healthcare professional working on the patient and other hospital personnel who may come into contact with it at a later time. For this reason, a significant amount of research and development has been performed on making such units safer to the various people who become exposed to them. This has spawned an entire new class of products which have various types of protection devices on the catheter emplacement unit in order to incapacitate the needle point such that it reduces the risk of sticking an individual after use.

Many of these devices require a modification of the introducer housing, that is, the actual portion of the introducer assembly gripped by the healthcare professional during the emplacement process. This housing usually has extending therefrom a cannula. Various types of devices have been attached to or passed through such housings in order to provide an attachment mechanism for protective features. This has made the fabrication of such housing a very complicated matter.

### Summary of the Invention

The present invention as defined in claim 1 provides a novel housing and method for making a housing such that the fabrication process is made easier and less expensive. In the fabrication process, the flash chamber which receives the blood flow back through the cannula during emplacement is fabricated separately from a least a portion of the remainder of the housing. This flash chamber is then attached to the housing in fluid communication with the cannula in order to provide the catheter emplacement unit. That is, the housing with flash chamber is the emplacement unit on which the catheter rides during its insertion into a patient. By forming the flash chamber as a separate piece and then attaching it to the housing, various modifications can be made to the housing in order to receive various aspects and portions of protective devices for protecting the healthcare professionals from accidental needle sticks. This simplifies the fabrication process as the molds are simpler to make as provision for the flash chamber need not be made in the housing mold and the later attachment of the flash chamber eases the fabrication process.

The flash chamber may be ultrasonically welded, adhesively attached, or press fit into the remainder of the housing. When the flash chamber is press fit into the remainder of the housing it is often advantageous to have formations on either the housing or the flash chamber to assist in the attachment of the flash chamber.

In a press-fit situation the housing may be provided with rings and the flash chamber may have an open end surrounded by ring-shaped barbs which are received within the rings of the housing. In this way the flash chamber, when press fit into the housing, causes the mating of the two sets of rings and an attachment of the flash chamber to the housing in a manner which reduces the risk of removal by a user.

### Brief Description of the Drawings

The invention will now be described with reference to the attached figures wherein
FIG. 1 shows a housing body for receiving a separate flash chamber;
FIG. 2 is a perspective view of a separately formed flash chamber for receipt in the housing of FIG. 1; and
FIG. 3 shows the assembled flash chamber and housing without a cannula.

### Description of the Preferred Embodiment

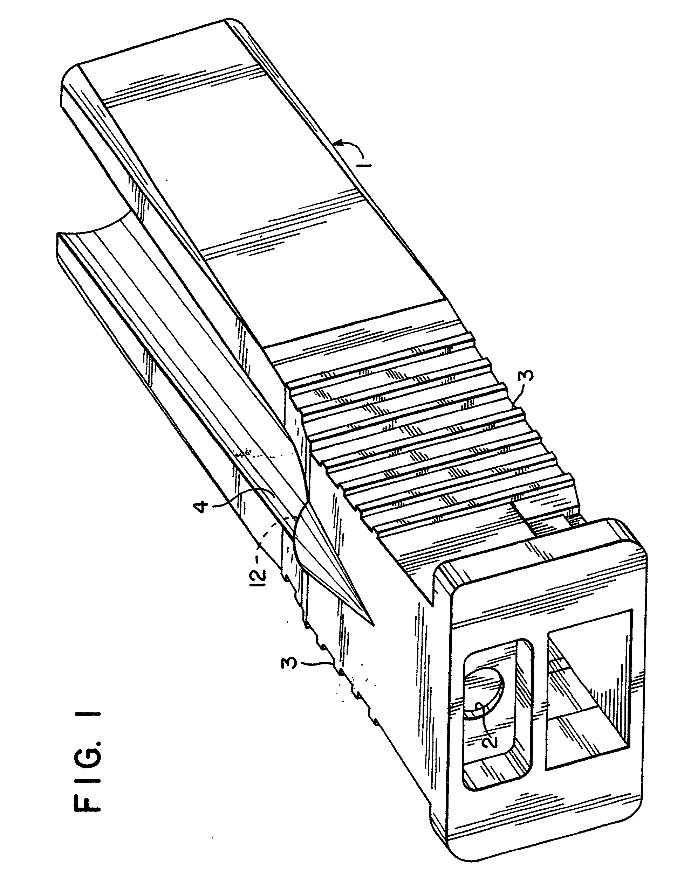

Referring to FIG. 1, there is shown a housing 1 having a cannula opening 2 defined in a front portion thereof. The cannula opening 2 is adapted for receipt of a hollow sharpened cannula (not shown) such that the cannula will extend from the housing for receipt of a catheter unit thereover.

The housing has a grip portion 3 having serrated edges for providing positive grip to the housing during emplacement of the device. A proximal hollow portion 4 is provided for receipt of a separate flash chamber which will be described hereafter. A distal portion of the hollow portion 4 defines an opening that is in fluid communication with the cannula opening 2.

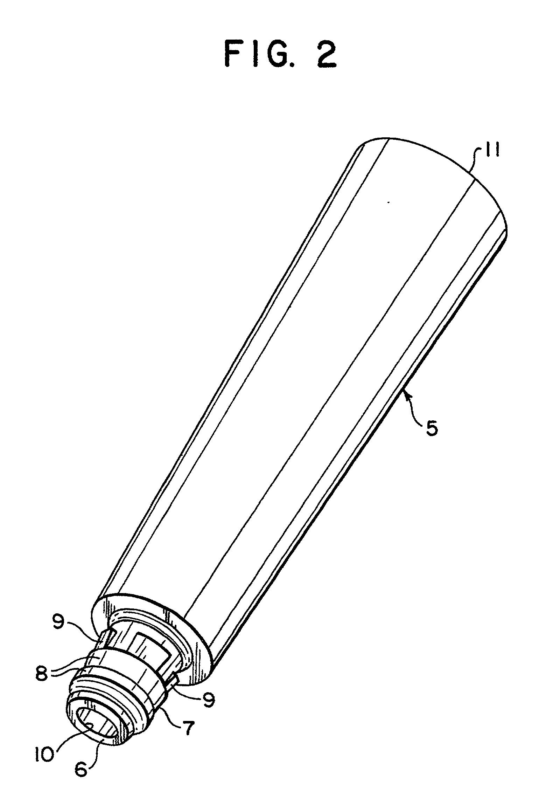

Referring now to FIG. 2, there is seen a flash chamber 5 separately formed from the housing of an appropriate material. The flash chamber is normally translucent or clear and therefore is formed out of a material such as polypropylene or polycarbonate in order to provide a visual indication of catheter emplacement by blood flowing into the flash chamber. A distal end 6 of the flash chamber 5 has formed thereon a neck 7. The neck 7 provides external rings 8 and barbs 9. A flash chamber opening 10 is defined by the neck 7 and is positioned at a distal end of the flash chamber. After fabrication of the flash chamber, the neck is inserted into the distal portion of the hollow portion 4 and press fit into the opening described above formed thereby. This opening has previously been described as in fluid communication with the cannula opening and therefore provides fluid communication between the cannula opening and the internal volume of the flash chamber 5. The flash chamber may have a second opening 11 at its proximal end for receipt of an appropriate porous flash plug (not shown). The flash plug permits the exit of air from the internal volume of the flash chamber and the replacement of the air by the blood flowing up the cannula. Otherwise, it is possible for the flash chamber to become airlocked and not permit the inflow of blood even though the catheter emplacement unit has properly positioned the catheter within the vein of a patient.

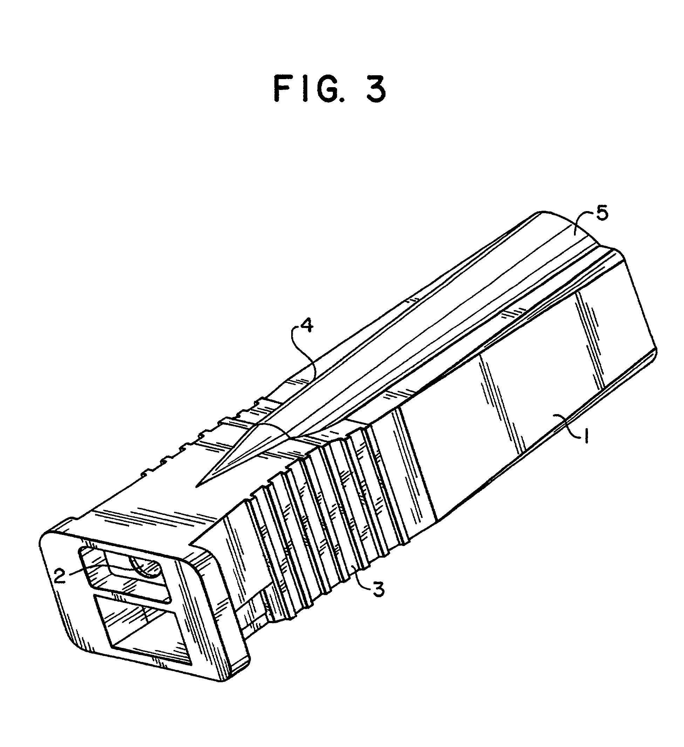

FIG. 3 shows the assembled flash chamber and housing unit. As is seen, the flash chamber 5 is received in the hollow portion 4 of the housing 1. After press fitting the flash chamber 5 presents a smooth outer surface in combination with the housing 1 to reduce the risk of gripping and removing of the flash chamber.

Although the flash chamber has been described with a neck 7 having external rings 8 and bars 9, it is easily seen that the same flash chamber may be made without such press fitting formations and separately ultrasonically welded to the housing or adhesively attached either through the neck or other means to the housing itself in a sealed condition. In this way, once attached, the flash chamber provides a unitary device with respect to blood flow from the tip of the sharpened cannula through to the flash chamber.

An attachment opening 12 has been previously formed in the housing 1 as, for example, during the molding of housing 1. The provision of a separate flash chamber eases the fabrication of the housing 1 with the attachment opening 12 defined therein. The gating and mold formation is simplified by the removal of the need to provide a mold which simultaneously forms the flash chamber. The attachment opening 12 is used to receive the attachment portion of a protective device such as a cap for capping the tip of the sharpened cannula after emplacement of the catheter unit. The attachment mechanism received in the attachment opening may, for example, be a sliding member that slides out of the housing as the capping portion of the device slides along the cannula during removal of the cannula from the catheter. The actual attachment mechanism has not been shown as it is anticipated that any number of the various types of attachment devices contemplated currently by those skilled in the art may be used with the present device.

## Claims

1. A catheter introducer assembly having:
a housing (1) for gripping by a user;
a cannula opening (2) in the housing;
a hollow sharpened cannula, extending from said housing (1) through said cannula opening (2), for piercing the skin of a patient during introduction of said catheter;
a flash chamber (5) which is separate from said housing and which is attached to said housing (1), said flash chamber (5) being in fluid communication with said hollow cannula for receiving blood that travels through said cannula during catheter introduction;
an attachment opening in said housing (1); and
a cannula protective device, the attachment portion of which is received in said attachment opening;
wherein:
said cannula is adapted to receive the catheter thereover; and
said flash chamber (5) attached to said housing (1) is obtainable by fabricating said flash chamber (5) and said housing (1) as separate pieces and attaching said separate pieces together by attachment means.

2. The assembly of claim 1 wherein said attachment means is a press fit between said flash chamber (5) and said housing (1).

3. The assembly of claim 2, wherein there is a formation (9) on said flash chamber (5) to assist in said attachment.

4. The assembly of claim 3 wherein said formation is a barbed information (9) on the flash chamber (5) for receipt within an opening defined by said housing (1).

5. The assembly of claim 1, wherein the attachment means comprises co-acting formations on the housing (1) and the flash chamber (5) to assist in attaching the flash chamber (5) to the housing (1).

6. The assembly of claim 5, wherein the formations assist in maintaining a seal between said flash chamber (5) and said housing (1).

7. A method for producing a catheter introducer assembly as defined in any one of claims 1 to 6 which comprises forming said housing (1) and said flash chamber (5) as separate pieces and thereafter attaching said flash chamber (5) to said housing (1) so that the flash chamber (5) is in fluid communication with said cannula.

8. The method of claim 7, wherein the flash chamber (5) is attached to the housing (1) by press fitting.

9. The method of claim 8, including the step of forming barbs (9) on a portion of said flash chamber (5) and wherein said press fitting fits said barbs (9) into a portion of said housing (1).

10. The method of any one of claims 7 to 9, further including the step of ultrasonically welding or thermally forming to attach said flash chamber (5) to said housing (1).

11. The method of claim 10, wherein said ultrasonic welding or thermal forming also seals said flash chamber (5) in communication with said cannula.

12. The method of any one of claims 7 to 11, further including the step of applying adhesive to at least one of said flash chamber (5) and said housing (1) to adhere said flash chamber (5) to said housing (1).

13. The method of claim 12, wherein said adhesive seals said flash chamber (5) in communication with said cannula.

## Patentansprüche

1. Kathetereinführungsanordnung mit:
einem Gehäuse (1) zum Ergreifen durch einen Anwender;
einer Kanülenöffnung (2) in dem Gehäuse;
einer hohlen, geschärften Kanüle, die sich von dem Gehäuse (1) durch die Kanülenöffnung (2) erstreckt, zum Durchstechen der Haut eines Patienten während des Einführens des Katheters;
einer Entspannungskammer (5), die von dem Gehäuse getrennt ist und die mit dem Gehäuse (1) verbunden ist, wobei die Entspannungskammer (5) in Fluidverbindung mit der hohlen Kanüle zum Aufnehmen von Blut steht, das während der Einführung des Katheters durch die Kanüle fließt;
einer Befestigungsöffnung in dem Gehäuse (1); und
einer Kanülenschutzvorrichtung, deren Befestigungsabschnitt in der Befestigungsöffnung aufgenommen ist;
wobei
die Kanüle ausgebildet ist, um den Katheter darüber aufzunehmen; und
die Entspannungskammer (5), die mit dem Gehäuse (1) verbunden ist, erhältlich ist durch Herstellen der Entspannungskammer (5) und des Gehäuses (1) als getrennte Teile und Verbinden der getrennten Teile durch Befestigungsmittel.

2. Anordnung nach Anspruch 1, wobei das Befestigungsmittel ein Presssitz zwischen der Entspannungskammer (5) und dem Gehäuse (1) ist.

3. Anordnung nach Anspruch 2, wobei auf der Entspannungskammer (5) eine Formation vorhanden ist, um das Verbinden zu unterstützen.

4. Anordnung nach Anspruch 3, wobei die Formation eine mit Widerhaken versehene Information (9) auf der Entspannungskammer (5) ist zur Aufnahme innerhalb einer durch das Gehäuse (1) definierten Öffnung.

5. Anordnung nach Anspruch 1, wobei das Befestigungsmittel miteinander wirkende Formationen auf dem Gehäuse (1) und der Entspannungskammer (5) umfasst, um das Verbinden der Entspannungskammer (5) mit dem Gehäuse (1) zu unterstützen.

6. Anordnung nach Anspruch 5, wobei die Formationen die Aufrechterhaltung einer Dichtung zwischen der Entspannungskammer (5) und dem Gehäuse ( 1 ) unterstützen.

7. Ein Verfahren zum Herstellen einer Kathetereinführungsanordnung, wie in einem der Ansprüche 1 bis 6 definiert, das Formen des Gehäuses (1) und der Entspannungskammer (5) als getrennte Teile und danach Verbinden der Entspannungskammer (5) mit dem Gehäuse ( 1 ), so dass die Entspannungskammer (5) in Fluidverbindung mit der Kanüle steht, umfasst.

8. Verfahren nach Anspruch 7, wobei die Entspannungskammer (5) mit dem Gehäuse (1) durch einen Presssitz verbunden ist.

9. Verfahren nach Anspruch 8, umfassend den Schritt der Ausbildung von Widerhaken (9) auf einem Abschnitt der Entspannungskammer (5) und wobei der Presssitz die Widerhaken (9) in einen Abschnitt des Gehäuses (1) einfügt.

10. Verfahren nach einem der Ansprüche 7 bis 9, weiter umfassend den Schritt des Ultraschallschweißens oder Heißformens, um die Entspannungskammer (5) mit dem Gehäuse (1) zu verbinden.

11. Verfahren nach Anspruch 10, wobei das Ultraschallschweißen oder Heißformen auch die Entspannungskammer (5) in Verbindung mit der Kanüle abdichtet.

12. Verfahren nach einem der Ansprüche 7 bis 11, weiter umfassend den Schritt des Auftragens von Klebstoff auf entweder die Entspannungskammern (5) oder das Gehäuse (1) oder beides, um die Entspannungskammer (5) mit dem Gehäuse (1) zu verkleben.

13. Verfahren nach Anspruch 12, wobei der Klebstoff die Entspannungskammer (5) in Verbindung mit der Kanüle versiegelt.

## Revendications

1. Ensemble d'introduction de cathéter, comprenant :
- un logement (1) susceptible d'être saisi par un utilisateur ;
- une ouverture de canule (2) ménagée dans le logement ;
- une canule aiguisée creuse, s'étendant depuis ledit logement (1) en traversant ladite ouverture de canule (2), pour percer la peau d'un patient au cours de l'introduction dudit cathéter ;
- une chambre de détente (5), séparée dudit logement et fixée sur ledit logement (1), ladite chambre de détente (5) étant en communication de fluide avec ladite canule creuse afin de recevoir du sang traversant ladite canule au cours de l'introduction du cathéter ;
- une ouverture de fixation ménagée dans ledit logement (1), et
- un dispositif de protection de canule, dont la partie de fixation est reçue dans ladite ouverture de fixation ;
- dans lequel :
- ladite canule est adaptée à recevoir le cathéter par-dessus celle-ci ; et
- ladite chambre de détente (5) fixée audit logement (1) est susceptible d'être obtenue en fabriquant ladite chambre de détente (5) et ledit logement (1) en tant que pièces séparées et en fixant lesdites pièces séparées ensemble par des moyens de fixation.

2. Ensemble selon la revendication 1, dans lequel lesdits moyens de fixation sont un ajustement serré entre ladite chambre de détente (5) et ledit logement (1).

3. Ensemble selon la revendication 2, dans lequel une conformation (9) est ménagée sur ladite chambre de détente (5) pour faciliter ladite fixation.

4. Ensemble selon la revendication 3, dans lequel ladite conformation (9) est une conformation à picots, ménagée sur la chambre de détente (5), susceptible d'être reçue à l'intérieur d'une ouverture définie par ledit logement (1).

5. Ensemble selon la revendication 1, dans lequel les moyens de fixation comprennent des conformations co-opérantes, ménagées sur le logement (1) et la chambre de détente (5), afin de faciliter la fixation de la chambre de détente (5) au logement (1).

6. Ensemble selon la revendication 5, dans lequel les conformations facilitent le maintien d'un joint entre ladite chambre de détente (5) et ledit logement (1).

7. Procédé de production d'un ensemble d'introduction de cathéter tel que défini dans l'une quelconque des revendications 1 à 6, comprenant la formation dudit logement (1) et de ladite chambre de détente (5) en tant que pièces séparées, puis la fixation de ladite chambre de détente (5) audit logement (1) de sorte que la chambre de détente (5) soit en communication de fluide avec ladite canule.

8. Procédé selon la revendication 7, dans lequel la chambre de détente (5) est fixée au logement (1) par un ajustement serré.

9. Procédé selon la revendication 8, comprenant une étape de formation de picots (9) sur une partie de ladite chambre de détente (5) et dans lequel ledit ajustement serré ajuste lesdits picots (9) dans une partie dudit logement (1).

10. Procédé selon l'une quelconque des revendications 7 à 9, comprenant, en outre, une étape de soudage aux ultrasons ou de conformation thermique pour effectuer la fixation de ladite chambre de détente (5) audit logement (1).

11. Procédé selon la revendication 10, dans lequel ledit soudage aux ultrasons ou ladite conformation thermique ménage également une étanchéité de ladite chambre de détente (5) en communication avec ladite canule.

12. Procédé selon l'une quelconque des revendications 7 à 11, comprenant, en outre, une étape d'application d'un adhésif sur au moins un des deux dispositifs, de ladite chambre de détente (5) et dudit logement (1), afin de faire adhérer ladite chambre de détente (5) audit logement (1).

13. Procédé selon la revendication 12, dans lequel ledit adhésif ménage également une étanchéité de ladite chambre de détente (5) en communication avec ladite canule.
